# EUROPEAN PATENT SPECIFICATION

(11) **EP 0 263 347 B1**
(45) Date of publication and mention of the grant of the patent: **13.04.1994**
(21) Application number: 87113813.7
(22) Date of filing: 22.09.1987
(51) Int. Cl.: A61B 17/06

(54) **Method of manufacturing a dark surgical needle**
Verfahren zur Herstellung einer dunklen chirurgischen Nadel
Procédé de fabrication d'une aiguille chirurgicale foncée

(30) Priority: 08.10.1986 US 916594
(43) Date of publication of application: 13.04.1988
(73) Proprietor: AMERICAN CYANAMID COMPANY, Wayne, NJ 07470-8426 (US)
(72) Inventor: Prasad, Janniah Srikanta, Norwalk Connecticut (US)
(74) Representative: Wächtershäuser, Günter, Prof. Dr.

(56) References cited:
- EP-A- 0 168 833
- FR-A- 2 055 297
- US-A- 3 210 220
- US-A- 3 963 530

## Description

This invention relates to a method of manufacturing a surgical needle having a dark, nonreflective and nonflaking surface.

A surgical needle having a dark and nonreflective surface is more visible at and around a wound site having a restrictive field of view, than a needle having a shiny and reflective stainless steel surface. That the dark and nonreflective surface of the needle must also be nonflaking is important, if not critical, so as not to introduce any foreign, extraneous, and possibly toxic substance into the wound.

Metallic product(s) having a dark, nonreflective and nonflaking surface may also be useful in diagnostic and analytical testing, for example where the absorption of an amount of light can be measured as a function of the increase in the temperature of the metallic product.

It is to be understood that the term "dark" in this specification is synonymous with the term "black", and further that the terms "dark", "black", "nonreflective" (or nonreflecting), and "nonflaking" in this specification have the same meaning as used in the prior art.

A surgical needle having a dark, nonreflective and nonflaking surface is known from EP-A-0168833. According to the reference the needle is produced by selecting an appropriately shaped needle having the desired degree of sharpness, treating the needle to activate the surface and then immersing it in a solution of sulfuric acid, potassium dichromate and water at a temperature in excess of 100 °C.

According to US-A-3210220 a color coating can be produced on a steel-chromium alloy surface by subjecting the surface to the action of a concentrated sulfuric or nitric acid water solution of chromium trioxide for a length of time necessary to form the desired thin oxide coating on said surface. The presence of tetrachromic acid in the process bath is believed to be responsable for the production of the thin oxide coating. The thickness and the color of the coating is dependent upon time of immersion, composition and temperature of the bath.

The present invention provides a method of manufacturing a surgical needle from a stainless steel, as defined in Claim 1.

The method comprises:
(a) selecting a stainless steel from, or substantially equal to a member of the group consisting of martensitic and ferritic steels, and forming the stainless steel selected into a needle having the desired configuration;
(b) submersing the needle into a solution of nitric acid and water;
(c) substantially maintaining a constant temperature of the solution while the needle is submersed;
(d) maintaining the needle in the constant temperature solution for a sufficient period of time to make the surface dark and nonreflective; and
(e) removing the needle from the constant temperature solution.

In one preferred embodiment, the method comprises the additional step of (f) neutralizing any residual and nonreacted acid remaining on the dark, nonreflective and nonflaking surface of the needle. In a specific embodiment, the neutralizing step comprises rinsing the dark, nonreflective and nonflaking surface of the needle with water; and drying the needle.

In another preferred embodiment, step (a) includes after forming the needle; removing any residual impurities selected from the group consisting of greases, oils and mixtures thereof remaining on the surface of the needle. In still another embodiment, the temperature of the solution in the substantially maintaining step is at about 82°C ± 5.5°C (180°F ± 10°F).

The solution in the submersing step may comprise about 50 percent by weight of nitric acid.

The neutralizing step may comprise: submersing the needle in a dilute base; first removing the product from the base; and second removing any residual and nonreacted base remaining on the dark, nonreflective and nonflaking surface of the needle. In a specific embodiment, the base is ammonium hydroxide.

The second removing sub-step of step (f) may comprise rinsing the dark, nonreflective and nonflaking surface of the surgical needle with a drying agent; and drying said needle. In a specific embodiment, the drying agent is ethanol.

### DESCRIPTION OF THE DRAWINGS

Figures 1 and 4 are microphotographs of two different dark, nonreflective and nonflaking metallic needles at 20 times magnification.
Figures 2 and 5 are microphotographs of the needles of Figures 1 and 3, respectively, at 100 times magnification;
Figures 3 and 6 are microphotographs of the needles of Figures 1 and 3, respectively, at 200 times magnification; and
Figure 7 is a graph showing the atomic oxygen content at the surface of a dark needle manufactured according to the method of this invention.

### DESCRIPTION OF THE PREFERRED EMBODIMENT(S)

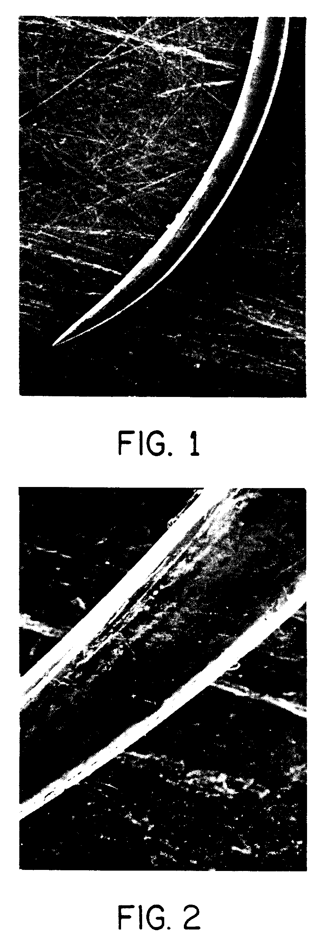

Figures 1 to 3 are microphotographs showing a dark, nonreflective and nonflaking surface of a metallic surgical needle. The microphotographs of Figures 1 to 3 have been taken at 20, 100, and 200 magnification, respectively.

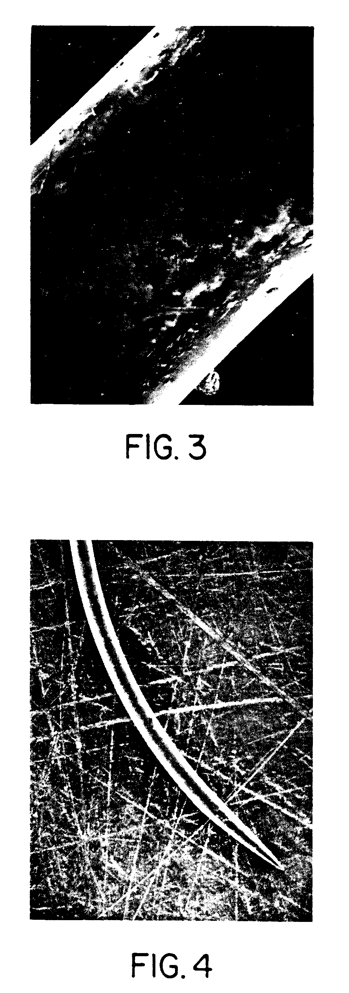

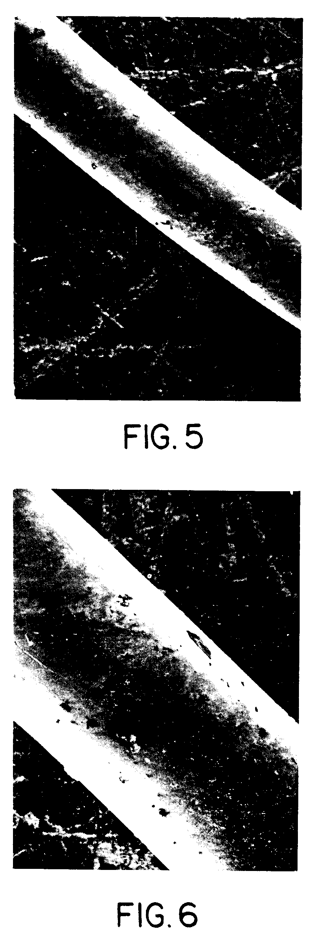

Figures 4 to 6 are microphotographs showing a dark, nonreflective and nonflaking surface of a different (from the needle of Figures 1 to 3) metallic surgical needle. As in Figures 1 to 3, the microphotographs of Figures 4 to 6 have been taken at 20, 100, and 200 magnification, respectively.

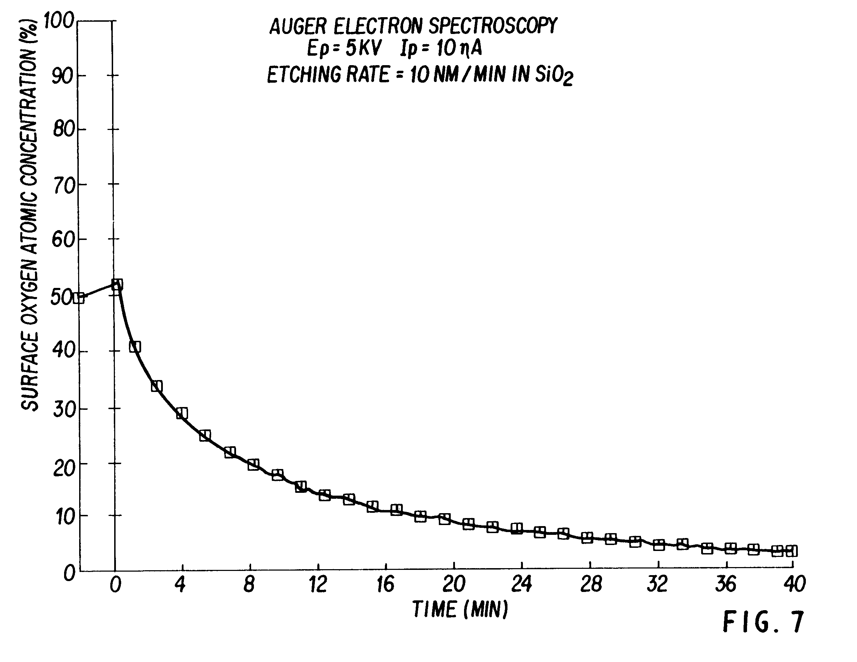

Figure 7 is a graph of the atomic oxygen content at the surface of a dark, nonreflective and nonflaking needle manufactured according to the method of this invention. The oxygen content is plotted against time (in minutes).

The curve of Figure 7 shows a smooth transition of the oxygen concentration over the time interval. That is, the smooth shape of the curve seems to suggest that the dark, nonreflective and nonflaking properties are even over the entire surface of the needle.

Surgical needles identical or similar to those shown in Figures 1 to 7 were submitted for toxicological review. With the submission, prior obtained information was provided indicating that the tissue penetration of surgical needles having a dark, nonreflective and nonflaking surface is essentially identical to a stainless steel surgical needle having a shiny and reflective surface; and that nothing comes off the dark and nonreflective surface (of the needles) when they are rubbed. Based on this prior information, and on an examination of electron microphotographs (similar or identical to those shown in Figures 1 to 6), a judgement was made that no toxicological evaluation of the surgical needles had to be conducted.

A solution is prepared containing about 50 percent nitric acid and 50 percent water. Tap water is satisfactory, although distilled water can also be used. The volume of the 50 percent nitric acid solution is not critical to the practice of the invention provided there is sufficient volume for the stainless steel needle(s) to be totally immersed in the solution.

The container holding the 50 percent nitric acid and water solution is heated to and maintained at a temperature of 82°C ± 5.5°C (180°F ± 10°F). The variance in the temperature (from 82°C (180°F)) is dependent on the boiling point of the nitric acid solution, the precision of the thermostat regulating the temperature, and the rate of reaction of the 50 percent nitric acid with the stainless steel needle(s).

A surgical needle manufactured from a material essentially identical to a type 420 or 420F martensitic stainless steel is immersed in the 50 percent nitric acid and water solution. The metallic needle is maintained in the 50 percent nitric acid and water solution until it turns dark.

The amount of time needed to turn the needle dark and nonreflective can be ascertained by the person skilled in the art without undue experimentation, and is not critical to the practice of this invention. Under many circumstances, a length of time of about 15 to 30 minutes can be considered normal. However, other times, for example from about 15 to 20 minutes, or up to about 40 minutes can also be used to adequately obtain a needle having a dark surface.

The needle is then removed from the nitric acid and water solution, and rinsed with water. Again, tap water can be used. Distilled water can also be used. Preferably, after rinsing any nitric acid remaining on the surface of the dark, nonreflective and nonflaking needle, it is neutralized by dipping it into a container holding dilute ammonium hydroxide.

If the preferred neutralizing step described above is carried out, the needle having a dark, nonreflective and nonflaking surface is rinsed again in tap water. The temperature of the tap water is about 38°C to 66°C (100°F to 150°F). The needle is then further rinsed with ethyl alcohol (as a drying agent) to remove any residual water remaining on the surface from the second water rinse.

It is important to note that ethyl alcohol is disclosed as the drying agent (for removing any residual water). However, ethyl alcohol is not critical to the practice of this invention. That is, any drying agent can be used provided it effectively removes the remaining residual water from the surface of the needle, and then evaporates off the surface or is nontoxic to living tissue.

The needle is then dried by any means known in the art, for example, air drying. The resulting surgical needle has a dark nonreflective and nonflaking surface.

## Claims

1. A method of manufacturing a surgical needle from a stainless steel, the surface of the needle being dark, nonreflective and nonflaking, comprising:
(a) selecting a stainless steel from, or substantially equal to a member of the group consisting of martensitic and ferritic steels, and forming the stainless steel selected into a needle having the desired configuration;
(b) submersing the needle into a solution of nitric acid and water;
(c) substantially maintaining a constant temperature of the solution while said needle is submersed;
(d) maintaining said needle in the constant temperature solution for a sufficient period of time to make the surface dark and nonreflective; and
(e) removing said needle from said constant temperature solution.

2. A method of claim 1 comprising the additional step of (f) neutralizing any residual and nonreacted acid remaining on the dark, nonreflective and nonflaking surface of the needle.

3. A method of claim 2 wherein the neutralizing step comprises rinsing the dark, nonreflective and nonflaking surface of the needle with water; and drying said needle.

4. A method of claim 3 wherein step (a) includes after forming the needle: removing any residual impurities selected from the group consisting of greases, oils and mixtures thereof remaining on the surface of the needle.

5. A method of claim 4 wherein the temperature of the solution in the substantially maintaining step is at about 82°C ± 5.5°C (180°F ± 10°F).

## Patentansprüche

1. Verfahren zum Herstellen einer chirurgischen Nadel aus einem korrosionsbeständigen Stahl, wobei die Oberfläche der Nadel dunkel, nicht reflektierend und nicht abblätternd ist, umfassend:
(a) Auswählen eines korrosionsbeständigen Stahls aus, oder im wesentlichen gleich, einem Glied der Gruppe, bestehend aus martensitischen und ferritischen Stählen und Formen des ausgewählten korrosionsbeständigen Stahls zu einer Nadel mit der erwünschten Konfiguration;
(b) Eintauchen der Nadel in eine Lösung aus Salpetersäure und Wasser;
(c) im wesentlichen Aufrechterhalten einer konstanten Temperatur der Lösung, während die Nadel eingetaucht ist;
(d) Halten der Nadel in der Lösung konstanter Temperatur für eine genügende Zeitdauer, um die Oberfläche dunkel und nicht reflektierend zu machen, und
(e) Herausnehmen der Nadel aus der Lösung konstanter Temperatur.

2. Verfahren nach Anspruch 1, umfassend die zusätzliche Stufe (f) des Neutralisierens irgendwelcher Rest- und unumgesetzten Säure, die auf der dunklen, nicht reflektierenden und nicht abblätternden Oberfläche der Nadel verblieben ist.

3. Verfahren nach Anspruch 2, worin die Neutralisationsstufe das Spülen der dunklen, nicht reflektierenden und nicht abblätternden Oberfläche der Nadel mit Wasser und das Trocknen der Nadel umfaßt.

4. Verfahren nach Anspruch 3, worin die Stufe (a) nach dem Formen der Nadel das Entfernen irgendwelcher Restverunreinigungen, ausgewählt aus der Gruppe bestehend aus Fetten, Ölen und deren Mischungen, die auf der Oberfläche der Nadel verblieben sind, einschließt.

5. Verfahren nach Anspruch 4, worin die Temperatur der Lösung im wesentlichen bei etwa 82°C ± 5,5°C (180°F ± 10°F) gehalten wird.

## Revendications

1. Procédé de fabrication d'une aiguille chirurgicale à partir d'acier inoxydable, la surface de l'aiguille étant sombre, non réfléchissante et sans écaille, comportant les étapes consistant à :
(a) choisir un acier inoxydable à partir d'un élément du groupe constitué d'aciers martensitique et ferritique ou d'un élément à peu près équivalent, et former l'acier inoxydable choisi sous forme d'une aiguille ayant la configuration voulue,
(b) immerger l'aiguille dans une solution d'acide nitrique et d'eau,
(c) maintenir la solution à une température pratiquement constante lorsque l'aiguille est immergée,
(d) maintenir l'aiguille dans la solution à température constante pendant une période de temps suffisante pour réaliser la surface sombre et non réfléchissante, et
(e) enlever l'aiguille de la solution à température constante.

2. Procédé selon la revendication 1, comportant l' étape supplémentaire (f) consistant à neutraliser tout acide résiduel et n'ayant pas réagi restant sur la surface sombre, non réfléchissante et sans écaille de l'aiguille.

3. Procédé selon la revendication 2, dans lequel l'étape de neutralisation comporte le rinçage de la surface sombre, non réfléchissante et sans écaille de l'aiguille à l'aide d'eau et le séchage de ladite aiguille.

4. Procédé selon la revendication 3, dans lequel l'étape (a) comporte après mise en forme de l'aiguille la suppression de toute impureté résiduelle constituée du groupe comportant les graisses, les huiles et les mélanges de ceux-ci, restant sur la surface de l'aiguille.

5. Procédé selon la revendication 4, dans lequel la température de la solution dans l'étape consistant à pratiquement maintenir la température est d'environ 82° C ± 5,5° C (180° F ± 10° F).
